# EUROPEAN PATENT SPECIFICATION

(11) **EP 1 199 355 B1**
(45) Date of publication and mention of the grant of the patent: **14.03.2007**
(21) Application number: 00913052.7
(22) Date of filing: 31.03.2000
(51) Int. Cl.: C12N 9/00, C12N 9/02, C12N 9/24, C12N 15/52, C12N 15/53, C12N 15/56

(54) **SULFUR ATOM-FREE ENZYME PROTEINS**
SCHWEFELFREIE ENZYME
PROTEINES ENZYMATIQUES EXEMPTES D'ATOMES DE SOUFRE

(30) Priority: 29.06.1999 JP 18366499
(43) Date of publication of application: 24.04.2002
(73) Proprietor: National Institute of Advanced Industrial Science and Technology, Tokyo 100-8921 (JP)
(72) Inventor: IWAKURA, Masahiro, Tsukuba-shi, Ibaraki 305-0035 (JP)
(74) Representative: Woods, Geoffrey Corlett
(86) International application number: PCT/JP2000/002112
(87) International publication number: WO 2001/000797

(56) References cited:
- EP-A1- 0 677 584
- EP-A2- 0 200 280
- EP-A2- 0 275 202
- EP-A2- 0 292 763
- EP-A2- 0 926 240
- WO-A1-95/12667
- ESTELL D A ET AL: "ENGINEERING AN ENZYME BY SITE-DIRECTED MUTAGENESIS TO BE RESISTANT TO CHEMICAL OXIDATION" JOURNAL OF BIOLOGICAL CHEMISTRY. (MICROFILMS), AMERICAN SOCIETY OF BIOLOGICAL CHEMISTS, BALTIMORE, MD, US, vol. 260, no. 11, 1985, pages 6518-6521, XP002900193
- IWAKURA MASAHIRO ET AL: "A strategy for testing the suitability of cysteine replacements in dihydrofolate reductase from Escherichia coli." JOURNAL OF BIOCHEMISTRY (TOKYO), vol. 117, no. 3, 1995, pages 480-488, XP009005575 ISSN: 0021-924X
- AMAKI YUSUKE ET AL: "Role of cysteine residues in esterase from Bacillus stearothermophilus and increasing its thermostable by the replacement of cysteines." APPLIED MICROBIOLOGY AND BIOTECHNOLOGY, vol. 40, no. 5, 1994, pages 664-668, XP009005576 ISSN: 0175-7598
- REDDY YETURU V R ET AL: "Probing the role of cysteine residues in the EcoP15I DNA methyltransferase." JOURNAL OF BIOLOGICAL CHEMISTRY, vol. 273, no. 37, pages 23866-23876, XP002231531 ISSN: 0021-9258
- CHEN P-F. ET AL.: 'Cysteine 184 of endothelial nitric oxide synthase is involved in heme coordination and catalytic activity' J. BIOL. CHEM., vol. 269, no. 40, 1994, pages 25062 - 25066, XP002929066
- MAGNUS J.H. ET AL.: 'Glycosaminoglycans in extracts of cardiac amyloid fibrils from familial amyloid cardiomyopathy of danish origin related to variant transthyretin Met III' SCAND. J. IMMUNOL., vol. 34, no. 1, 1991, pages 63 - 69, XP002929067
- YOSHIMOTO T. ET AL.: 'Pyroglutamyl peptidase gene from Bacillus amyloliquefaciens: cloning, sequencing, expression and crystaallization of the expressed enzyme' J. BIOCHEM., vol. 113, no. 1, 1993, pages 67 - 73, XP002929068

### SEQUENCE LISTING

<110> NATIONAL INSTITUTE OF ADVANCED INDUSTRIAL SCIENCE AND TECHNOLOGY
<120> SULFUR ATOM-FREE ENZYME PROTEIN
<130> N.84517 GCW
<140> 00913052.7
   <141> 2000-03-31
<150> JP99/183664
   <151> 1999-06-29
<160> 10
<170> Patent In Ver. 2.0
<210> 1
   <211> 159
   <212> PRT
   <213> E. coli
<400> 1
   Met Ile Ser Leu Ile Ala Ala Leu Ala Val Asp Arg Val Ile Gly
   1 5 10 15
   Met Glu Asn Ala Met Pro Trp Asn Leu Pro Ala Asp Leu Ala Trp
   20 25 30
   Phe Lys Arg Asn Thr Leu Asn Lys Pro Val Ile Met Gly Arg His
   35 40 45
   Thr Trp Glu Ser Ile Gly Arg Pro Leu Pro Gly Arg Lys Asn Ile
   50 55 60
   Ile Leu Ser Ser Gln Pro Gly Thr Asp Asp Arg Val Thr Trp Val
   65 70 75
   Lys Ser Val Asp Glu Ala Ile Ala Ala Ala Gly Asp Val Pro Glu
   80 85 90
   Ile Met Val Ile Gly Gly Gly Arg Val Tyr Glu Gln Phe Leu Pro
   95 100 105
   Lys Ala Gln Lys Leu Tyr Leu Thr His Ile Asp Ala Glu Val Glu
   110 115 120
   Gly Asp Thr His Phe Pro Asp Tyr Glu Pro Asp Asp Trp Glu Ser
   125 130 135
   Val Phe Ser Glu Phe His Asp Ala Asp Ala Gln Asn Ser His Ser
   140 145 150
   Tyr Ser Phe Glu Ile Leu Glu Arg Arg
   155
<210> 2
   <211> 566
   <212> DNA
   <213> E. coli
<221> CDS
   <222> (81)...(557)
<400> 2
   ggatccttga caattagtta actatttgtt ataatgtatt catgagctta actaactaat
   ccggaaaagg aggaacttcc atgatcagtc tgattgcggc gctagcggta gatcgcgtta
   tcggcatgga aaacgccatg ccatggaacc tgcctgccga tctcgcctgg tttaaacgca
   acaccttaaa taaacccgtg attatggggc gccatacctg ggaatcaatc ggtaggcctt
   tgcccggccg caaaaatatt atcctcagca gtcaacccgg gaccgatgat cgggttacct
   gggttaaatc ggtcgacgaa gccatcgcgg ccgcaggtga cgtaccagaa atcatggtga
   ttggcggcgg acgcgtttat gaacagttct tgccaaaagc gcaaaagctt tatctgacgc
   atatcgatgc agaagtggaa ggcgacaccc attttccgga ttacgagccg gatgactggg
   aatcggtatt cagcgaattc cacgatgctg atgcgcagaa ctcgcatagc tattcgttcg
   aaatcctcga gcgtcgttaa ggatcc
<210> 3
   <211> 185
   <212> PRT
   <213> B. subtilis
<400> 3
   Ala Ser Thr Asp Tyr Trp Gln Asn Trp Thr Asp Gly Gly Gly Ile
   1               5                   10                  15
   Val Asn Ala Val Asn Gly Ser Gly Gly Asn Tyr Ser Val Asn Trp
                   20                  25                  30
   Ser Asn Thr Gly Asn Phe Val Val Gly Lys Gly Trp Thr Thr Gly
                   35                  40                  45
   Ser Pro Phe Arg Thr Ile Asn Tyr Asn Ala Gly Val Trp Ala Pro
                   50                  55                  60
   Asn Gly Asn Gly Tyr Leu Thr Leu Tyr Gly Trp Thr Arg Ser Pro
                   65                  70                  75
   Leu Ile Glu Tyr Tyr Val Val Asp Ser Trp Gly Thr Tyr Arg Pro
                   80                  85                  90
   Thr Gly Thr Tyr Lys Gly Thr Val Lys Ser Asp Gly Gly Thr Tyr
                   95                  100                 105
   Asp Ile Tyr Thr Thr Thr Arg Tyr Asn Ala Pro Ser Ile Asp Gly
                   110                 115                 120
   Asp Arg Thr Thr Phe Thr Gln Tyr Trp Ser Val Arg Gln Ser Lys
                   125                 130                 135
   Arg Pro Thr Gly Ser Asn Ala Thr Ile Thr Phe Ser Asn His Val
                   140                 145                 150
   Asn Ala Trp Lys Ser His Gly Met Asn Leu Gly Ser Asn Trp Ala
                   155                 160                 165
   Tyr Gln Val Met Ala Thr Glu Gly Tyr Gln Ser Ser Gly Ser Ser
                   170                 175                 180
   Asn Val Thr Val Trp
                   185
<210> 4
   <211> 558
   <212> DNA
   <213> B. subtilis
<221> CDS
   <222> (1)...(555)
<400> 4
   gctagcacag actactggca aaattggact gatgggggcg gtatagtaaa cgctgtcaat
   gggtctggcg ggaattacag tgttaattgg tctaataccg gaaattttgt tgttggtaaa
   ggttggacta caggttcgcc atttaggacg ataaactata atgccggagt ttgggcgccg
   aatggcaatg gatatttaac tttatatggt tggacgagat cacctctcat agaatattat
   gtagtggatt catggggtac ttatagacct actggaacgt ataaaggtac tgtaaaaagt
   gatgggggta catatgacat atatacaact acacgttata acgcaccttc cattgatggc
   gatcgcacta cttttacgca gtactggagt gttcgccagt cgaagagacc aaccggaagc
   aacgctacaa tcactttcag caatcatgtg aacgcatgga agagccatgg aatgaatctg
   ggcagtaatt gggcttacca agtcatggcg acagaaggat atcaaagtag tggctcgtcg
   aatgttaccg tatggtaa
<210> 5
   <211> 159
   <212> PRT
   <213> E. coli
<400> 5
   Ala Ile Ser Leu Ile Ala Ala Leu Ala Val Asp Arg Val Ile Gly
   1 5 10 15
   Asn Glu Asn Ala Leu Pro Trp Asn Leu Pro Ala Asp Leu Ala Trp
   20 25 30
   Phe Lys Arg Asn Thr Leu Asn Lys Pro Val Ile Tyr Gly Arg His
   35 40 45
   Thr Trp Glu Ser Ile Gly Arg Pro Leu Pro Gly Arg Lys Asn Ile
   50 55 60
   Ile Leu Ser Ser Gln Pro Gly Thr Asp Asp Arg Val Thr Trp Val
   65 70 75
   Lys Ser Val Asp Glu Ala Ile Ala Ala Ala Gly Asp Val Pro Glu
   80 85 90
   Ile Phe Val Ile Gly Gly Gly Arg Val Tyr Glu Gln Phe Leu Pro
   95 100 105
   Lys Ala Gln Lys Leu Tyr Leu Thr His Ile Asp Ala Glu Val Glu
   110 115 120
   Gly Asp Thr His Phe Pro Asp Tyr Glu Pro Asp Asp Trp Glu Ser
   125 130 135
   Val Phe Ser Glu Phe His Asp Ala Asp Ala Gln Asn Ser His Ser
   140 145 150
   Tyr Ser Phe Glu Ile Leu Glu Arg Arg
   155
<210> 6
   <211> 569
   <212> DNA
   <213> E. coli
<221> CDS
   <222> (81)...(560)
<400> 6
   ggatccttga caattagtta actatttgtt ataatgtatt catgagctta actaactaat
   ccggaaaagg aggaacttcc atggcaatca gtctgattgc ggcgctagcg gtagatcgcg
   ttatcggcaa cgaaaacgcc ctcccatgga acctgcctgc cgatctcgcc tggtttaaac
   gcaacacctt aaataaaccc gtgatttacg ggcgccatac ctgggaatca atcggtaggc
   ctttgcccgg ccgcaaaaat attatcctca gcagtcaacc cgggaccgat gatcgggtta
   cctgggttaa atcggtcgac gaagccatcg cggccgcagg tgacgtacca gaaatcttcg
   tgattggcgg cggacgcgtt tatgaacagt tcttgccaaa agcgcaaaag ctttatctga
   cgcatatcga tgcagaagtg gaaggcgaca cccattttcc ggattacgag ccggatgact
   gggaatcggt attcagcgaa ttccacgatg ctgatgcgca gaactcgcat agctattcgt
   tcgaaatcct cgagcgtcgt taaggatcc
<210> 7
   <211> 353
   <212> PRT
   <213>
<400> 7
   Ala Ile Ser Leu Ile Ala Ala Leu Ala Val Asp Arg Val Ile Gly
   1 5 10 15
   Asn Glu Asn Ala Leu Pro Trp Asn Leu Pro Ala Asp Leu Ala Trp
   20 25 30
   Phe Lys Arg Asn Thr Leu Asn Lys Pro Val Ile Tyr Gly Arg His
   35 40 45
   Thr Trp Glu Ser Ile Gly Arg Pro Leu Pro Gly Arg Lys Asn Ile
   50 55 60
   Ile Leu Ser Ser Gln Pro Gly Thr Asp Asp Arg Val Thr Trp Val
   65 70 75
   Lys Ser Val Asp Glu Ala Ile Ala Ala Ala Gly Asp Val Pro Glu
   80 85 90
   Ile Phe Val Ile Gly Gly Gly Arg Val Tyr Glu Gln Phe Leu Pro
   95 100 105
   Lys Ala Gln Lys Leu Tyr Leu Thr His Ile Asp Ala Glu Val Glu
   110 115 120
   Gly Asp Thr His Phe Pro Asp Tyr Glu Pro Asp Asp Trp Glu Ser
   125 130 135
   Val Phe Ser Glu Phe His Asp Ala Asp Ala Gln Asn Ser His Ser
   140 145 150
   Tyr Ser Phe Glu Ile Leu Glu Arg Arg Gly Gly Gly Gly Ser Gly
   155 160 165
   Gly Gly Gly Ala Ser Thr Asp Tyr Trp Gln Asn Trp Thr Asp Gly
   170 175 180
   Gly Gly Ile Val Asn Ala Val Asn Gly Ser Gly Gly Asn Tyr Ser
   185 190 195
   Val Asn Trp Ser Asn Thr Gly Asn Phe Val Val Gly Lys Gly Trp
   200 205 210
   Thr Thr Gly Ser Pro Phe Arg Thr Ile Asn Tyr Asn Ala Gly Val
   215 220 225
   Trp Ala Pro Asn Gly Asn Gly Tyr Leu Thr Leu Tyr Gly Trp Thr
   230 235 240
   Arg Ser Pro Leu Ile Glu Tyr Tyr Val Val Asp Ser Trp Gly Thr
                       245                 250                 255
   Tyr Arg Pro Thr Gly Thr Tyr Lys Gly Thr Val Lys Ser Asp Gly
                       260                 265                 270
   Gly Thr Tyr Asp Ile Tyr Thr Thr Thr Arg Tyr Asn Ala Pro Ser
                       275                 280                 285
   Ile Asp Gly Asp Arg Thr Thr Phe Thr Gln Tyr Trp Ser Val Arg
                       290                 295                 300
   Gln Ser Lys Arg Pro Thr Gly Ser Asn Ala Thr Ile Thr Phe Ser
                       305                 310                 315
   Asn His Val Asn Ala Trp Lys Ser His Gly Met Asn Leu Gly Ser
                       320                 325                 330
   Asn Trp Ala Tyr Gln Val Met Ala Thr Glu Gly Tyr Gln Ser Ser
                       335                 340                 345
   Gly Ser Ser Asn Val Thr Val Trp
                       350
<210> 8
   <211> 1153
   <212> DNA
   <213>
<400> 8
   ggatccttga caattagtta actatttgtt ataatgtatt catgagctta actaactaat
   ccggaaaagg aggaacttcc atggcaatca gtctgattgc ggcgctagcg gtagatcgcg
   ttatcggcaa cgaaaacgcc ctcccatgga acctgcctgc cgatctcgcc tggtttaaac
   gcaacacctt aaataaaccc gtgatttacg ggcgccatac ctgggaatca atcggtaggc
   ctttgcccgg ccgcaaaaat attatcctca gcagtcaacc cgggaccgat gatcgggtta
   cctgggttaa atcggtcgac gaagccatcg cggccgcagg tgacgtacca gaaatcttcg
   tgattggcgg cggacgcgtt tatgaacagt tcttgccaaa agcgcaaaag ctttatctga
   cgcatatcga tgcagaagtg gaaggcgaca cccattttcc ggattacgag ccggatgact
   gggaatcggt attcagcgaa ttccacgatg ctgatgcgca gaactcgcat agctattcgt
   tcgaaatcct cgagcgtcgt ggtggcggtg gctcgggtgg tggcggcgct agcacagact
   actggcaaaa ttggactgat gggggcggta tagtaaacgc tgtcaatggg tctggcggga
   attacagtgt taattggtct aataccggaa attttgttgt tggtaaaggt tggactacag
   gttcgccatt taggacgata aactataatg ccggagtttg ggcgccgaat ggcaatggat
   atttaacttt atatggttgg acgagatcac ctctcataga atattatgta gtggattcat
   ggggtactta tagacctact ggaacgtata aaggtactgt aaaaagtgat gggggtacat
   atgacatata tacaactaca cgttataacg caccttccat tgatggcgat cgcactactt
   ttacgcagta ctggagtgtt cgccagtcga agagaccaac cggaagcaac gctacaatca
   ctttcagcaa tcatgtgaac gcatggaaga gccatggaat gaatctgggc agtaattggg
   cttaccaagt catggcgaca gaaggatatc aaagtagtgg ctcgtcgaat gttaccgtat
   ggtaaggatc ccc
<210> 9
   <211> 353
   <212> PRT
   <213>
<400> 9
   Ala Ile Ser Leu Ile Ala Ala Leu Ala Val Asp Arg Val Ile Gly
   1 5 10 15
   Asn Glu Asn Ala Leu Pro Trp Asn Leu Pro Ala Asp Leu Ala Trp
   20 25 30
   Phe Lys Arg Asn Thr Leu Asn Lys Pro Val Ile Tyr Gly Arg His
   35 40 45
   Thr Trp Glu Ser Ile Gly Arg Pro Leu Pro Gly Arg Lys Asn Ile
   50 55 60
   Ile Leu Ser Ser Gln Pro Gly Thr Asp Asp Arg Val Thr Trp Val
   65 70 75
   Lys Ser Val Asp Glu Ala Ile Ala Ala Ala Gly Asp Val Pro Glu
   80 85 90
   Ile Phe Val Ile Gly Gly Gly Arg Val Tyr Glu Gln Phe Leu Pro
   95 100 105
   Lys Ala Gln Lys Leu Tyr Leu Thr His Ile Asp Ala Glu Val Glu
   110 115 120
   Gly Asp Thr His Phe Pro Asp Tyr Glu Pro Asp Asp Trp Glu Ser
   125 130 135
   Val Phe Ser Glu Phe His Asp Ala Asp Ala Gln Asn Ser His Ser
   140 145 150
   Tyr Ser Phe Glu Ile Leu Glu Arg Arg Gly Gly Gly Gly Ser Gly
   155 160 165
   Gly Gly Gly Ala Ser Thr Asp Tyr Trp Gln Asn Trp Thr Asp Gly
   170 175 180
   Gly Gly Ile Val Asn Ala Val Asn Gly Ser Gly Gly Asn Tyr Ser
   185 190 195
   Val Asn Trp Ser Asn Thr Gly Asn Phe Val Val Gly Lys Gly Trp
   200 205 210
   Thr Thr Gly Ser Pro Phe Arg Thr Ile Asn Tyr Asn Ala Gly Val
   215 220 225
   Trp Ala Pro Asn Gly Asn Gly Tyr Leu Thr Leu Tyr Gly Trp Thr
   230 235 240
   Arg Ser Pro Leu Ile Glu Tyr Tyr Val Val Asp Ser Trp Gly Thr
   245 250 255
   Tyr Arg Pro Thr Gly Thr Tyr Lys Gly Thr Val Lys Ser Asp Gly
   260 265 270
   Gly Thr Tyr Asp Ile Tyr Thr Thr Thr Arg Tyr Asn Ala Pro Ser
   275 280 285
   Ile Asp Gly Asp Arg Thr Thr Phe Thr Gln Tyr Trp Ser Val Arg
   290 295 300
   Gln Ser Lys Arg Pro Thr Gly Ser Asn Ala Thr Ile Thr Phe Ser
   305 310 315
   Asn His Val Asn Ala Trp Lys Ser His Gly Leu Asn Leu Gly Ser
   320 325 330
   Asn Trp Ala Tyr Gln Val Ile Ala Thr Glu Gly Tyr Gln Ser Ser
   335 340 345
   Gly Ser Ser Asn Val Thr Val Trp
   350
<210> 10
   <211> 1153
   <212> DNA
   <213>
<400>10
   ggatccttga caattagtta actatttgtt ataatgtatt catgagctta actaactaat
   ccggaaaagg aggaacttcc atggcaatca gtctgattgc ggcgctagcg gtagatcgcg
   ttatcggcaa cgaaaacgcc ctcccatgga acctgcctgc cgatctcgcc tggtttaaac
   gcaacacctt aaataaaccc gtgatttacg ggcgccatac ctgggaatca atcggtaggc
   ctttgcccgg ccgcaaaaat attatcctca gcagtcaacc cgggaccgat gatcgggtta
   cctgggttaa atcggtcgac gaagccatcg cggccgcagg tgacgtacca gaaatcttcg
   tgattggcgg cggacgcgtt tatgaacagt tcttgccaaa agcgcaaaag ctttatctga
   cgcatatcga tgcagaagtg gaaggcgaca cccattttcc ggattacgag ccggatgact
   gggaatcggt attcagcgaa ttccacgatg ctgatgcgca gaactcgcat agctattcgt
   tcgaaatcct cgagcgtcgt ggtggcggtg gctcgggtgg tggcggcgct agcacagact
   actggcaaaa ttggactgat gggggcggta tagtaaacgc tgtcaatggg tctggcggga
   attacagtgt taattggtct aataccggaa attttgttgt tggtaaaggt tggactacag
   gttcgccatt taggacgata aactataatg ccggagtttg ggcgccgaat ggcaatggat
   atttaacttt atatggttgg acgagatcac ctctcataga atattatgta gtggattcat
   ggggtactta tagacctact ggaacgtata aaggtactgt aaaaagtgat gggggtacat
   atgacatata tacaactaca cgttataacg caccttccat tgatggcgat cgcactactt
   ttacgcagta ctggagtgtt cgccagtcga agagaccaac cggaagcaac gctacaatca
   ctttcagcaa tcatgtgaac gcatggaaga gccatggact caatctgggc agtaattggg
   cttaccaagt catcgcgaca gaaggatatc aaagtagtgg ctcgtcgaat gttaccgtat
   ggtaaggatc ccc

## Description

### FIELD OF THE INVENTION

The present invention relates to methods of producing a sulfur atom-free enzyme protein having activity greater than or equivalent to the original enzyme protein from which the sulfur atom-free enzyme protein is derived.

### BACKGROUND ART

Enzymes, because of their very high substrate specificity, has been attempted and expected to be used, for example, in development of analytical instruments such as biosensors, in fine chemical industries such as bioreactors, and in decomposition and removal of specific pollutants.

Recent development of mass spectroscopes has enabled mass spectrometry of proteins. When determining the mass number of proteins by mass spectrometry, even in proteins that are highly purified as proteins, existence of proteins having a mass number other than that expected from the amino acid sequence has been found. For example, as a result of detailed inspection using L-cystein-free dihydrofolate reductase, it was found that the change in mass number was based on a unit of the mass number 16, and the main cause thereof is oxidation of methionine.

When considering the utilization of enzymes, denaturation of proteins caused by oxidation becomes a serious drawback. For example, many biosensors make use of an electrode reaction, and in this case, oxides such as hydrogen peroxide are produced due to the electrode reaction. The oxides could oxidize enzymes, thereby accelerating deterioration of the entire sensor or reducing reliability. When an enzyme is used in a solution for a long period of time, the prevention of oxidation by oxygen in an aqueous solution is difficult or problematic from a viewpoint of cost.

There are 5 types of atom which constitute proteins: hydrogen (H); carbon (C); nitrogen (N); oxygen (O); and sulfur (S). Among them, the sulfur atom has greater reactivity such as valence properties, in comparison with other atoms.

Proteins produced by organisms are constituted by 20 types of L-amino acid residues: L-alanine; L-aspartic acid; L-glutamic acid; L-phenylalanine; L-glycine; L-histidine; L-isoleucine; L-lysine; L-leucine; L-asparagine; L-proline; L-glutamine; L-arginine; L-serine; L-threonine; L-valine; L-tyrosine; L-tryptophan; L-cystein; and L-methionine; which are coded by triplet codons in DNA of genes. Among them, L-cystein and L-methionine are sulfur-containing amino acids.

The sulfur atom of L-cystein exists as a thiol (-SH) group. The thiol group has a very high reactivity, is easily oxidized by oxygen and hydrogen peroxide, and produces disulfide, and further sulfinic acid.

The sulfur atom of L-methionine exists as a thioether (-SCH3) group. The thioether group has reactivity not as strong as that of the thiol group, however, it is easily oxidized by hydrogen peroxide to produce methionine sulfoxide.

This suggests that an enzyme which does not include L-cystein and L-methionine, i.e., one which is free from sulfur-containing amino acids, possesses antioxidative properties.

There are 3 types of codon corresponding to L-cystein and L-methionine, that is, TGT and TGC (L-cystein) and ATG (L-methionine). In contrast, there are 58 types of codon corresponding to other 18 types of sulfur-free L-amino acid. Therefore, proteins contain L-cystein or L-methionine at a ratio of 1 in every 20 amino acids (3/61) on average. This indicates that proteins constituted by 100 or more amino acids contain L-cystein or L-methionine with a very high probability. In fact, among the proteins which have hitherto been reported, when enzymes having catalytic functions are inspected, enzymes free from L-cystein and L-methionine are not found.

That is, all enzymes produced by organisms contain sulfur-containing amino acids.

On the other hand, results of protein mutation based on recent genetic manipulation demonstrate that at least in the case of mutation of one site, it is possible to substitute sulfur-containing amino acids by other amino acids without loss of original enzyme function. Before the present invention was completed, it had not been clarified whether or not enzymes, in which all sulfur-containing amino acids were substituted with other amino acids, had activities equivalent to the original functions. Rather, it was the dominant point of view that sulfur-containing amino acids were essential to expression of enzyme activity.

This suggests the following two opposing possibilities.

The first possibility is that the presence of the sulfur-containing amino acids is essential to the development of very high enzyme activity necessary for organisms to maintain their lives.

The second possibility is that the presence of the sulfur-containing amino acids is not necessarily essential to the development of very high enzyme activity. However, in the origins of life, proteins had happened to be utilized which contained sulfur atoms therein and the form for coding genes was established, and thus, enzymes produced by organisms contain sulfur-containing amino acids corresponding to the frequency with which the codon was used. Therefore, enzymes can be prepared which all the sulfur-containing amino acids are substituted with other amino acids, and which have equivalent functions to organism-produced enzymes containing sulfur-containing amino acids.

If the second possibility were correct, a general process for preparing enzymes having antioxidation properties can be developed wherein all the sulfur-containing amino acids of enzymes which contain organism-derived sulfur-containing amino acids are substituted with other amino acids.

### DISCLOSURE OF THE INVENTION

The present inventors have keenly studied and substantiated that, an enzyme which has enzyme activity equivalent to or greater than the original enzyme, can be prepared even where all sulfur-containing amino acids of an enzyme which contains organism-derived sulfur-containing amino acids are substituted with other amino acids. The present inventors further established a strategy to vary an organism-derived wild type enzyme to an enzyme free from sulfur-containing amino acids. This has led to the completion of the present invention.

According to the present invention, a wild type enzyme includes enzymes obtained by artificially varying organism-derived enzymes in addition to the organism-derived enzymes.

More specifically, the object of the present invention is to provide a method for producing an enzyme protein which retains the activity of a wild type enzyme while having an antioxidation property against oxidation by hydrogen peroxide and the like.

The present invention therefore provides a method of producing a sulfur atom-free enzyme protein having activity greater than or equivalent to the original enzyme protein from which said sulfur atom-free enzyme protein is derived, comprising the following steps:
(1) preparing respective mutant genes by substituting an initiation codon encoding L-methionine in a DNA sequence encoding an enzyme protein which consists of a total length of m amino acids and which comprises n sulfur atom-containing amino acids (sulfur-containing amino acids), wherein the respective sulfur-containing amino acids in the sequence are designated Ai (i = 1 to n), by codons for L-methionine-L-alanine, L-methionine-L-serine, or L-methionine-L-proline; expressing each mutant gene in a host cell; measuring enzyme activity of each obtained mutant enzyme protein; and selecting the protein with the highest activity, thereby obtaining a substitution mutant A1/MA1;
(2) preparing respective mutant genes in which a codon encoding a sulfur-containing amino acid at another site Ai (i = 2 to n) is substituted with a codon encoding another amino acid selected from L-alanine, L-aspartic acid, L-glutamic acid, L-phenylalanine, L-glycine, L-histidine, L-isoleucine, L-lysine, L-leucine, L-asparagine, L-proline, L-glutamine, L-arginine, L-serine, L-threonine, L-valine, L-tyrosine, and L-tryptophan; expressing each mutant gene in a host cell; measuring the enzyme activity of each obtained mutant enzyme protein; and selecting p mutant enzyme proteins having enzyme activity, thereby obtaining substitution mutants Ai/Bij (j = 1 to p);
(3) selecting from the p mutant enzyme proteins produced in (2) a maximum of 3 substitution mutants having the highest activity Ai/Bil, Ai/Bi2, and Ai/Bi3, wherein activity decreases in order, Ai/Bil>Ai/Bi2>Ai/Bi3>..>Ai/Bip; and
(4) selecting according to steps (2) and (3) for all sites Ai (i = 2 to n) a maximum of 3 substitution mutants having activity; preparing a maximum of 3 x (n-1) mutants which constitute all combinations of the selected substitution mutants with the mutant of A1/MA1, measuring the enzyme activity thereof; and obtaining a mutant enzyme protein having activity greater than or equivalent to the original enzyme protein (a process for producing an enzyme free from sulfur containing amino acids by combined mutation)

The present invention further provides a method of producing a sulfur atom-free enzyme protein having activity greater than or equivalent to the original enzyme protein from which said sulfur atom-free enzyme protein is derived, comprising the following steps:
(1) preparing respective mutant genes by substituting an initiation codon encoding L-methionine in a DNA sequence encoding an enzyme protein which consists of a total length of m amino acids and which comprises n sulfur atom-containing amino acids (sulfur-containing amino acids), wherein the respective sulfur-containing amino acids in the sequence are designated Ai (i = 1 to n), by codons for L-methionine-L-alanine, L-methionine-L-serine, or L-methionine-L-proline; expressing each mutant gene in a host cell; measuring enzyme activity of each obtained mutant enzyme protein; and selecting the protein with the highest activity, thereby obtaining a substitution mutant A1/MA1;
(2) preparing respective mutant genes in which a codon encoding a sulfur-containing amino acid at position A2 of the A1/MA1 mutant is substituted with a codon encoding another amino acid selected from L-alanine, L-aspartic acid, L-glutamic acid, L-phenylalanine, L-glycine, L-histidine, L-isoleucine, L-lysine, L-leucine, L-asparagine, L-proline, L-glutamine, L-arginine, L-serine, L-threonine, L-valine, L-tyrosine and L-tryptophan; expressing each mutant gene in a host cell; measuring the enzyme activity of each obtained double mutant enzyme protein; and selecting a maximum of 3 double mutants with the highest activity;
(3) preparing respective mutant genes in which a codon encoding a sulfur-containing amino acid at position A3 of each obtained double mutant is substituted with a codon encoding another amino acid selected from L-alanine, L-aspartic acid, L-glutamic acid, L-phenylalanine, L-glycine, L-histidine, L-isoleucine, L-lysine, L-leucine, L-asparagine, L-proline, L-glutamine, L-arginine, L-serine, L-threonine, L-valine, L-tyrosine and L-tryptophan; expressing each mutant gene in a host cell; measuring the enzyme activity of each obtained triple mutant enzyme protein; and selecting a maximum of 3 triple mutants with the highest activity; and
(4) preparing mutants in the same manner as described above at each further site of a sulfur-containing amino acid up to site n; inspecting the enzyme activity of the mutants produced at site n; and selecting a mutant enzyme protein having activity greater than or equivalent to that of the original enzyme (a process for producing an enzyme free from sulfur-containing amino acids by stepwise mutation).

The present invention also provides a method for producing a sulfur atom-free enzyme protein by stepwise mutation as described above wherein the order of stepwise mutation sites is according to any one of n! permutations and combinations of A1, A2, ..., An.

The present invention also provides a method for producing a sulfur atom-free enzyme protein having activity greater than or equivalent to the original enzyme protein from which said sulfur atom-free enzyme protein is derived, wherein, in an enzyme protein consisting of a total length of m amino acids and comprising n sulfur-containing amino acids and in which the position of a sulfur-containing amino acid in the sequence is designated Ai (i = 1 to n), the method for producing an enzyme free from sulfur containing amino acids by combined mutation as described above is adopted at k sites and the method for producing an enzyme free from sulfur containing amino acids by stepwise mutation as described above is adopted at n - k sites wherein k is a natural number less than n.

In the present invention, to retain activity of the original proteins means that the protein does not have less than 10% activity, preferably not less than 50% activity, particularly preferably not less than 100% activity, of the wild type enzyme, and can be usable for the same applications as the original enzyme proteins.

The procedure of the first method to prepare the intended enzyme protein in the present invention will be described.

It is provided that a wild type enzyme consisting of a total length of m amino acids contains n sulfur-containing amino acids. The respective positions of the sulfur-containing amino acids in the amino acid sequence are designated as Ai (i = 1 to n).

The amino acid resulting from the initiation codon of a protein is L-methionine.

To avoid amino terminal L-methionine, methionine can be eliminated from the amino terminus pursuant to the reaction specificity of methionine-aminopeptidase found in the host cell.

The present invention provides a method as described above wherein the host cell is *Escherichia coli.* When *Escherichia coli* is employed as a host, any of L-methionine-L-alanine, L-methionine-L-serine and L-methionine-L-proline may be adopted as the amino terminus so that a protein is expressed with eliminating L-methionine from the terminal. Accordingly, at the outset, as a mutation of the amino terminus of the enzyme, mutant genes having codons for L-methionine-L-alanine, L-methionine-L-serine, or L-methionine-L-proline are prepared, the prepared mutant genes are expressed in a host, the activity of the obtained mutants are measured, and the mutant with the highest activity level is selected to prepare a mutant enzyme in which the amino terminus is not methionine. The mutation thus obtained is represented by A1/MA1.

Regarding sulfur-containing amino acids at other sites Ai (i = 2 to n), the codon encoding the sulfur-containing amino acid is substituted with a codon encoding an "amino acid other than a sulfur-containing amino acid" (a maximum of 18 types) to prepare one or more mutant genes. Each obtained mutant gene is expressed in a host cell, and the obtained double mutant enzyme proteins are inspected for enzyme activity.

The present invention also provide a method wherein amino acid substitution at a specific site in an enzyme protein is carried out by site-directed mutagenesis using synthetic DNA.

As methods for site-directed mutagenesis, many methods are known including a method by Zoller, Smith et al. (Zoller, M. J. and Smith, M. (1983) Methods in Enzymology, vol. 100, p. 468) and improvements thereof, and methods utilizing PCR employed in the present examples and the like. In the present invention, any mutation method may be adopted so far as it can be applied to amino acid substitution of a sulfur-containing amino acid at a target amino acid site, and that object can be accomplished. Therefore, the present invention is not limited by the method for preparing mutants

When a substitution mutant of sulfur-containing amino acid Ai is prepared to inspect its activity, p mutants exhibiting activity equivalent to or greater than the wild type enzyme are found. The amino acid is determined as Bij (j = 1 to p) where activity of Ai/Bij substitution mutant is ordered from highest to lowest. More specifically, activities of the mutants decrease in the order of Ai/Bi1>Ai/Bi2>Ai/Bi3> ·· >Ai/Bip.

Among Ai/Bij substitution mutants, a maximum of 3 substitution mutants having the highest activity are selected, specifically, Ai/Bi1,Ai/Bi2, and Ai/Bi3.

Selection is carried out in the same manner for all i (i=2 to n) to prepare a maximum of 3 x (n - 1) mutants which constitute all combinations of these mutations with mutation A1/MA1. The activities of 3 x (n - 1) mutants are then inspected to select the mutant having activity equivalent to or greater than that of the wild type enzyme.

It is obvious that the mutant enzymes thus obtained are free of sulfur-containing amino acids.

The enzymes free from sulfur-containing amino acid thus obtained having activity equivalent to or greater than the wild type enzyme are insusceptible to oxidation by treatment with hydrogen peroxide, etc.

The first method is referred to as "a method for producing an enzyme free from sulfur-containing amino acids by combined mutation".

An enzyme free from sulfur-containing amino acids having activity equivalent to or greater than the wild type enzyme can be also prepared in accordance with the following second method.

A1/MA1 mutant is prepared in accordance with the first method.

Subsequently, one or more double mutants (a maximum of 18 types) are respectively prepared in the same manner as described above wherein the sulfur-containing amino acid at A2 of A1/MA1 mutant is substituted with one or more amino acids other than sulfur-containing amino acid (a maximum of 18 types). Enzyme activities are then inspected.

When inspecting the activities of the double mutants, mutants having activities equivalent to or greater than that of the wild type are found. From among the double mutants, a maximum of 3 double mutants having the highest activity are selected.

Triple mutants are then prepared respectively (a maximum of 3 x 18 = 54 types) by substituting sulfur-containing amino acids at position A3 of each obtained double mutant by one or more amino acids other than sulfur-containing amino acids (a maximum of 18 types). Enzyme activities thereof are inspected.

When inspecting the activities of the triple mutants, mutants with activity equivalent to or greater than that of the wild type are found.

In the same manner as described above, mutants are prepared at each further site of a sulfur-containing amino acid up to site n. The mutant produced at site n is the contemplated enzyme, which does not contain a sulfur-containing amino acid.

For convenience of description, the order of the sites to be varied is represented as A1, A2,..., An, however, the order of mutation may be selected from any one of n! permutations and combinations.

The second method is referred to as "a method for producing an enzyme free from sulfur-containing amino acids by stepwise mutation".

In the "method for producing an enzyme free from sulfur-containing amino acids by stepwise mutation", a maximum of [4(A1) + 18 (A2) + 54 x (n-2) (A3 to An)] mutants are inspected.

An enzyme not containing a sulfur-containing amino acid and having activity equivalent to or greater than that of a wild type enzyme may obviously be prepared by "the method for producing an enzyme free from sulfur-containing amino acids by combined mutation" in partial combination with "the method for producing an enzyme free from sulfur-containing amino acids by stepwise mutation" (referred to as "a method for producing an enzyme free from sulfur-containing amino acids by combined mutation in combination with stepwise mutation").

Information on the amino acid sequence and the base sequence of the target wild type enzyme is sufficient for preparing sulfur atom-free enzyme proteins. For example, in accordance with information on the base sequence, a PCR primer is synthesized. DNA encoding the wild type enzyme can be synthesised by PCR using DNA of cells producing the wild type enzyme, cDNA, or recombinant plasmid DNA as a template. DNA encoding the wild type enzyme can also be prepared by chemical synthesis on the basis of the base sequence. The present invention is performed by subjecting DNA encoding the wild type enzyme to mutation in accordance with the method of the present invention. Accordingly, the present invention is not limited by the gene of the wild type enzyme.

The examples of the present invention provides a typical example of an oxidoreductase in which a cysteine free mutant (abbreviated as AS-DHFR) of *Escherichia coli*-derived dihydrofolate reductase (abbreviated as DHFR) is used as a starting material, 5 methionines contained in AS-DHFR are substituted with other amino acids to prepare DHFR free from sulfur-containing amino acids, having high activity far exceeding the enzyme activity of the wild type DHFR which contains 2 cysteine residues and 5 methionine residues. Thus, the present invention provides a method as described above wherein the sulfur atom-free enzyme protein is *E. coli*-derived dihydrofolate reductase. As a typical example of a hydrolytic enzyme, preparation of xylanase free from sulfur-containing amino acids, which has activity equivalent to the wild type enzyme is illustrated, in which 2 methionines contained in *Bacillus subtilis*-derived xylanase are substituted with other amino acids. Thus, the invention also provides a method as described above wherein the sulfur atom-free enzyme protein is *B. subtilis*-derived xylanase.

SEQ ID NO 1 in the Sequence Listing shows an amino acid sequence of AS-DHFR, SEQ ID NO 2 in the Sequence Listing shows a gene sequence which can be excised with restriction enzyme BamHI and is highly expressible in *Escherichia coli* by introducing into the BamHI site of an appropriate vector for *Escherichia coli*, SEQ ID NO 3 in the Sequence Listing shows the amino acid sequence of Bacillus subtilis-derived xylanase, and SEQ ID NO 4 of the Sequence Listing shows a DNA sequence encoding *Bacillus*, *subtilis-*derived xylanase.

DNA of SEQ ID NO 2 in the Sequence Listing can be prepared by using a recombinant plasmid, pTZDHFR20, having a sequence reported by the present inventors (described in Journal of Biochemistry vo1.117, p.480-488 (1995)) as a template by employing PCR using two primer DNAs, for example, 5'-ggatccttgacaattagttaactat-3' and 5'-ggatccttaacgacgctcgaggattt-3'. The DNA can also be prepared by a chemical synthesis based on the sequence of SEQ ID NO 2.

DNA of SEQ ID NO 4 in the Sequence Listing can be prepared from chromosome DNA of *Bacillus subtilis* by employing PCR using, for example, a primer DNA indicated by 5'-gctagcacag actactggcaaaat-3' and 5'-ttaccatacggtaacattcgacg-3'. The present invention employs DNA isolated using chromosome DNA (product number D4041, commercially available from Sigma) as a chromosome DNA of *Bacillus subtilis*. The DNA can also be prepared by a chemical synthesis based on the sequence of SEQ ID NO 4.

AS-DHFR comprises 159 amino acids. Among those, the 1st, 16th, 20th, 42nd and methionine is substituted with another amino acid, the Examples describe a process employing "a method for producing an enzyme free from sulfur-containing amino acids by a combination of combined mutation and stepwise mutation".

Tables 1 to 5 show the enzyme activities of mutants in which the 1st, 16th, 20th, 42nd, and 92nd methionines of AS-DHFR are substituted with other amino acids, Table 6 shows the enzyme activities of mutants in which the 42nd and 92nd methionines of AS-DHFR are substituted with other amino acids, and Table 7 shows enzyme activities of mutants in which the 16th and 20th methionines of AS-DHFR are substituted with other amino acids.

Regarding the 1st methionine, three types of mutant were prepared in accordance with the above process. As a result, methionine-alanine selected as the optimal one (see Table 1). This mutant was named AS-DHFR-A1.

Regarding the 16th, 20th, 42nd, and 92nd methionines of AS-DHFR, a random bases- substituted mutant was prepared using a primer with a sequence in which each ATG, as a codon for methionine, had been replaced with NNY (N represents a base of A, T, G, and C and Y represents a base of T and C). The base sequence and the enzyme activity of the obtained mutant were measured. As a result, alanine, phenylalanine, and asparagine were determined as preferable 16th amino acids; isoleucine, leucine, and valine were determined as preferable 20th acids; valine and tyrosine were determined as preferable 42nd amino acids; and phenylalanine and isoleucine were determined as preferable 92nd amino acids (see Tables 2 to 5).

Next, valine and tyrosine as the preferable 42nd amino acids are combined with phenylalanine and isoleucine as the preferable 92nd amino acids to prepare the 42nd and 92nd amino acid substitution mutants of AS-DHFR-A1. As a result of measurement of the base sequence and the enzyme activity of the obtained mutants, a combination of tyrosine as the 42nd amino acid and phenylalanine as the 92nd amino acid exhibited the greatest activity (about three times greater than the wild type enzyme). This mutant was named AS-DHFR-A1-M42Y-M92F (see Table 6).

Subsequently, alanine, phenylalanine, and asparagine as preferable 16th amino acids were combined with isoleucine, leucine, and valine as preferable 20th amino acids to prepare 16th and 20th amino acid substitution mutants of AS-DHFR-A1-M42Y-M92F. The base sequence and the enzyme activity of the obtained mutants were measured. As a result, as shown in Table 7, mutant enzymes, the activity of which exceeded that of the wild type enzyme, was obtained. Among them, the combination of asparagine as the 16th amino acid and leucine as the 20th amino acid exhibited the greatest activity (about twelve times greater than the wild type enzyme). This mutant was named ANLYF (based on the single letter representation of the mutations at each methionine site MIMA, M16N, M20L, M42Y, and M92F of AS-DHFR. The same representation is employed hereinafter).

A total of nine mutants including AFLYF, AFIYF, ANIYF, AFVYF, and ANVYF were obtained as sulfur atom-free mutants having activity greater than the wild type enzyme in addition to ANLYF.

This result indicates that there are many possibilities for sulfur atom-free enzyme proteins. Conversion of the wild type enzyme sequence in accordance with the method of the present invention can prepare a sulfur atom-free enzyme having activity greater than the wild type enzyme.

Xylanase comprises 185 amino acids with the 158th and the 169th amino acids being methionine. As a method for preparing enzymes in which methionine is substituted with other amino acids, in the Examples, a method is described wherein "a method for producing an enzyme free from sulfur-containing amino acids by stepwise mutation" is employed.

Specifically, a fusion protein was prepared wherein a carboxy terminal of the ANLYF and an amino terminal of xylanase were linked with a sequence, Gly-Gly-Gly-Gly-Ser-Gly-Gly-Gly-Gly (this was named NL-xylanase). Neither the ANLYF portion in NL-xylanase nor the linker region contained methionine and cystein Fusion with ANLYF allows selection of transformants into which NL-xylanase mutant fusion genes have been introduced on the basis of trimethoprim resistance and by measuring xylanase activity and DHFR activity and calculating the value [xylanase activity]/[DHFR activity], it is possible to compare activity of mutant xylanase with wild type xylanase activity without measuring the protein amount of NL-mutant xylanase.

A random bases-substituted mutant was prepared using a primer having a sequence in which "atg" as a codon for the 158th methionine in the xylanase portion of NL-xylanase was changed to "nny" (n represents a base for a, c, g, t and y represents a base for t, c). The base sequence and enzyme activity of the prepared mutant were measured and as a result, a mutant, which had been substituted to leucine, exhibited 127% activity of the wild type. This mutant was named NL-xylanase (M158L)

Subsequently, a random bases-substituted mutant was prepared using a primer having a sequence in which "atg" as a codon for the 168th methionine in xylanase portion of NL-xylanase (M158L) was changed to "nny" (n represents a base for a, c, g, t and y represents a base for t, c). The base sequence and the enzyme activity of the prepared mutant were measured. As a result, a mutant, which had been substituted to isoleucine, exhibited 151 % activity of the wild type. This mutant was denoted NL-xylanase (M158L, M169I).

Neither the DHFR portion, the linker region, nor the xylanase of the obtained NL-xylanase (M158L, M169I) contained the sulfur-containing amino acids, cystein or methionine. In spite of that, the enzyme activity of DHFR and that of xylanase had greater activities than corresponding wild type enzymes respectively.

DHFR is a so-called, oxidoreductase which catalyzes an oxidation-reduction reaction requiring a nicotinamide coenzyme. Xylanase is a so-called hydrolytic enzyme which catalyzes a hydrolysis reaction of high molecular polysaccharides. Two examples of the present invention demonstrate that, in enzymes catalyzing completely different reactions, i.e., an oxidation-reduction reaction and a hydrolysis reaction, enzymes can be prepared with activity exceeding that of wild type enzymes produced by organic evolution, containing no sulfur-containing amino acids at all. This indicates the existence of proteins having activity exceeding or equivalent to the activity of a wild type enzyme in proteins wherein all the sulfur-containing amino acids contained in the wild type enzyme are substituted with other amino acids. The present invention provides an assured method for being led to such enzymes free from sulfur-containing amino acid.

Dihydrofolate reductase catalyzes the reaction represented by the following reaction formula 1 :

dihydrofolic acid + NADPH → tetrahydrofolic acid + NADP+ (reaction formula 1).

Activity of dihydrofolate reductase can be traced by a decrease in absorbance at 340 nm caused by a decrease in substrate caused by reaction. In the method described in Example 1, the enzyme reaction solution comprises 50 mM phosphate buffer (pH 7), 0.1 mM NADPH, 0.05 .mM dihydrofolic acid, 12 mM 2-mercaptoetanol, and a suitable amount of enzyme. One ml of enzyme reaction solution was collected in a cuvette for a spectrophotometer. A reaction was initiated with the addition of the enzyme solution and change in amount of absorbance at 340 nm per minute was measured. The quantity was taken as an indicator of activity.

Xylanase catalyzes the reaction represented by the following reaction formula 2 :

xylan+n H₂O→ m-xylose oligomer (reaction formula 2).

The activity of xylanase can be observed by measuring an increase of reducing power derived from a reducing terminus of the prepared oligoxylose in accordance with the Neison-Somogyi method. In the method described in Example 2, the enzyme reaction solution comprises 50 mM sodium acetate buffer (pH 6.0), 2 mg/ml oat derived-xylane, and a proper amount of enzyme. The enzyme reaction solution (0.25 ml) was poured into a test tube. A reaction was initiated with the addition of the enzyme solution and the reaction was performed for 10 min. After 0.25 ml of copper-alkali reagent was added, the reaction solution was retained in boiled water for 10 min and then cooled to room temperature. Thereafter, 0.25 ml of arsenic- molybdic acid reagent was added, thereby coloring the solution. The intensity of coloring was measured by absorbance at 500 nm to determine a difference between the measured value and the absorbance of the control sample. The obtained value was adopted as an index for enzyme activity.

It is well known that various processes are developed and used for measuring enzyme activity. Thus, the present invention is obviously not limited by a process for measuring the subject enzyme activity.

The sulfur atom-free dihydrofolate reductase and a fused protein of dihydrofolate reductase-xylanase prepared according to the present invention do not contain in the sequences thereof, cystein and methionine which are easily oxidized by hydrogen peroxide. Therefore, treatment with 0.1 M highly concentrated hydrogen peroxide water does not result in oxidation, and resistance against oxidation is significantly enhanced. For either dihydrofolate reductase or a fused protein of dihydrofolate reductase-xylanase, in the case of a sulfur atom-containing enzyme, treatment with 0.1 M hydrogen peroxide water oxidizes all the sulfur molecules, thereby lowering the enzyme activity to about one tenth or below. It is verified that enhancement of an antioxidative property in this manner contributes to the stability of enzyme traits.

### BEST MODE TO CARRY OUT THE INVENTION

The present invention will be described with reference to the following examples, though the present invention is not limited to these specific examples only.

In the examples, DNA amplification by PCR, DNA cleavage by restriction enzymes, T4-DNA ligase, DNA linkage by transformation to Escherichia coli were carried out in accordance with a standard protocol attached to a commercially available PCR kit, restriction enzymes, T4-DNA ligase, and Escherichia coli competent cells.

### [Example 1] Preparation of sulfur atom-free dihydrofolate reductase

In the present example, AS-DHFR was used as a sulfur atom-containing dihydrofolate reductase. Properties of AS-DHFR such as stability of enzyme activity are substantially in conformity with the wild type enzyme (described in M. Iwakura, B.E. Jones, J. Luo, & C.R. Matthews, J. Biochemistry, 117, 480-488 (1995)).

The amino acid sequence of AS-DHFR and the base sequence of genes thereof are shown in SEQ. ID NO 1 in the Sequence Listing and SEQ. ID NO 2 in the Sequence Listing, respectively.

Genes of AS-DHFR are incorporated in a plasmid which is named "pTZDHFR20" (described in M. Iwakura, B.E. Jones, J. Luo, & C.R. Matthews, J. Biochemistry, 117,480-488 (1995)).

Two chemically-synthesized primer DNAs: 5'-ggggatcctcttgacaattagttaactatttgttataatgtattc-3' and 5'-ggggatcccttatgcacagccaccgccaccacgacgctcgaggatttcg-3', were used, and amplification performed by PCR using pTZDHFR20 as a template, thereby preparing a gene sequence which is able to be cleaved by a restriction enzyme BamHI and to express AS-DHFR (DNA1)

The DNA sequence amplified by PCR was used as a template to prepare genes wherein methionine as the 1st amino acid was varied to L-methionine-L-alanine, L-methionine-L-serine, or L-methionine-L-proline. In order to prepare the genes, DNA amplified by PCR using two chemically-synthesized primer DNAs: 5'-ggggatcctcttgacaattagttaactatttgttataatgtattc-3' and 5'-cgcaatcagactgatngncatggaagttcctccttttccggatt-3' (n represents a base of a, c, g or t) (DNA2) and DNA, amplified by PCR using two chemically-synthesized primer DNAs: 5'-ggaggaacttccatgncnatcagtctgattgcggcgctagcggtagat-3' (n represents a base of a, c, g or t) and 5'-ggggatcccttatgcacagccaccgccaccacgacgctcgaggatttcg-3' (DNA3) were prepared, and then, DNA2 was mixed with DNA3 in equal amounts, followed by preparation of DNA amplified by PCR using two chemically-synthesized primer DNAs: 5'-ggggatcctcttgacaattagttaactatttgttataatgtattc-3' and 5'-ggggatcccttatgcacagccaccgccaccacgacgctcgaggatttcg-3' (DNA4).

DNA4 was cleaved by BamHI, and then combined with a commercially available plasmid vector pUC19 which had been cleaved by BamHI, and they were ligated using T4-DNA ligase to prepare a recombinant plasmid. The obtained recombinant plasmid was used to transform Escherichia coli JM109 strain and a mutant growing in agar medium 1 (containing 5 g of salt, 5 g of yeast extract, 8 g of trypton, 100 mg of sodium ampicillin, 50 mg of trimethoprim, and 15 g of agar, per liter) was selected. In respect of 15 colonies formed in agar medium 1, plasmids were separated, and the base sequences thereof were determined, thereby isolating a transformant comprising a gene in which methionine as the 1st amino acid had been varied to L-methionine-L-alanine, L-methionine-L-serine, or L-methionine-L-proline. Each of the isolated transformants was cultured at 37 degree overnight in medium 1 (containing 5 g of salt, 5 g of yeast extract, 8 g of trypton, and 100 mg of ampicillin, per liter). Absorbance at 660 nm was adjusted to 1, bacterial cells were collected from 1 ml of the culture solution, suspended in 0.2 ml 10 mM phosphate buffer (pH 7.0), subjected to sonication, and then centrifuged. The obtained supernatant was inspected for its DHFR enzyme activity. The results are shown in Table 1.

In the Table below, DHFR activity is indicated by % with the activity of the wild type enzyme being 100%.

**Table I**

| (Types of mutation) | (DHFR activity) |
|---|---|
| L-methionine-L-alanine | 103 |
| (AS-DHFR-A1) | |
| L-methionine-L-serine | 98 |
| L-methionine-L-proline | 89 |

The results indicate that L-methionine-L-alanine is suitable as a mutant for the 1st amino acid (this mutant is referred to as AS-DHFR-A1, and a recombinant plasmid including AS-DHFR-A1 gene is referred to as pAS-DHFR-A1). AS-DHFR-A1 was highly purified, and the sequence of the amino terminus was inspected. As a result, 99% or more of the amino acids were alanine, and methionine resulting from the initiation codon on the gene sequence had been substantially completely excised by methionyl-aminopeptidase during expression in Escherichia coli.

Regarding the 16th, 20th, 42nd, and 92nd methionines of AS-DHFR, a random bases-substituted mutant was prepared using a primer having a sequence in which atg, a codon of methionine, has been varied to nny (n represents a base of a, c, g or t, r represents a base of a or g, and y represents a base of c or t). The base sequence and the enzyme activity of the obtained mutant were measured.

In preparation of a mutant for the 16th methionine, by using DNA1 as a template, DNA amplified by PCR using two chemically-synthesized primer DNAs: 5'-ggggatcctcttgacaattagttaactatttgttataatgtattc-3' and 5'-ggcatggcgttttcrnngccgataacgcgatctaccgcta-3' (DNA5) and DNA amplified by PCR using two chemically-synthesized primer DNAs: 5'-gatcgcgttatcggcnnygaaaacgccatgccatggaac-3' and 5'-ggggatcccttatgcacagccaccgccaccacgacgctcgaggatttcg-3' (DNA6) were prepared. Subsequently, DNA5 was mixed with the same amount of DNA6 and then, DNA amplified by PCR using two chemically-synthesized primer DNAs: 5'-ggggatcctcttgacaattagttaactatttgttataatgtattc-3' and 5'-ggggatcccttatgcacagccaccgccaccacgacgctcgaggatttcg-3' (DNA7) was prepared. After cleaving DNA7 with BamHI, the same procedures as described above were performed and colonies growing in agar medium 1 were inspected. The results are shown in Table 2.

**Table 2**

| [Types of mutation] | [DHFR activity] |
|---|---|
| (16th amino acid) | |
| L-alanine | 99 |
| L-aspartic acid | 9.5 |
| L-phenylalanine | 234 |
| glycine | 31 |
| L-histidine | 44 |
| L-isoleucine | 52 |
| L-leucine | 30 |
| L-asparagine | 100 |
| L-proline | 1.7 |
| L-arginine | 60 |
| L-serine . | 95 |
| L-threonine | 61 |
| L-valine | 34 |
| L-tyrosine. | 73 |

This result indicates that alanine, phenylalanine, and asparagine are suitable as the 16th amino acid.

In preparation of a mutant for the 20th methionine, using DNA1 as a template, DNA amplified by PCR using two chemically-synthesized primer DNAs: 5'-ggggatcctcttgacaattagttaactatttgttataatgtattc-3' and 5'-aggcaggttccatggrnnggcgttttccatgccgataac-3' (DNA8) and DNA amplified by PCR using two chemically-synthesized primer DNAs : 5'-ggcatggaaaacgccnnyccatggaacctgcctgccgatc-3' and 5'-ggggatcccttatgcacagccaccgccaccacgacgctcgaggatttcg-3' (DNA9) were prepared. Subsequently, DNA8 was mixed with the same amount of DNA9 and then, DNA amplified by PCR using two chemically-synthesized primer DNAs: 5'-ggggatcctcttgacaattagttaactatttgttataatgtattc-3' and 5'-ggggatcccttatgcacagccaccgccaccacgacgctcgaggatttcg-3' (DNA 10) was prepared. After cleaving DNA 10 with BamHI, the same procedures as described above were performed and colonies growing in agar medium 1 were inspected. The results are shown in Table 3.

**Table 3**

| [Types of mutation] | [DHFR activity] |
|---|---|
| (20th amino acid) | |
| L-aspartic acid | 3.7 |
| glycine | 0.8 |
| L-histidine | 1.7 |
| L-isoleucine | 182 |
| L-leucine | 283 |
| L-proline | 1.2 |
| L-arginine | 2 |
| L-serine | 20 |
| L-threonine | 63 |
| L-valine | 122 |
| L-tyrosine | 46 |

This result indicates that isoleucine, leucine, and valine are suitable as the 20th amino acid.

In preparation of a mutant for the 42nd methionine, by using DNA 1 as a template, DNA amplified by PCR using two chemically-synthesized primer DNAs: 5'-ggggatcctcttgacaattagttaactatttgttataatgtattc-3' and 5'-ccaggtatggcgcccmnaatcacgggtttatttaaggtg-3' (DNA11) and DNA amplified by PCR using two chemically-synthesized primer DNAs: 5'-aataaacccgtgattnnygggcgccatacctgggaatcaa-3' and 5'-ggggatcccttatgcacagccaccgccaccacgacgctcgaggatttcg-3' (DNA 12) were prepared. Subsequently, DNA 11 was mixed with the same amount of DNA 12 and then, DNA obtained through amplification by PCR using two chemically-synthesized primer DNAs: 5'-ggggatcctcttgacaattagttaactatttgttataatgtattc -3' and 5'-ggggatcccttatgcacagccaccgccaccacgacgctcgaggatttcg-3' (DNA13) was prepared. After cleaving DNA 13 with BamHI, the same procedures as described above were performed and colonies growing in agar medium 1 were inspected. The results are shown in Table 4.

**Table 4**

| [Types of mutation] | [DHFR activity] |
|---|---|
| (42nd amino acid) | |
| L-alanine | 63 |
| L-phenylalanine | 49 |
| glycine | 5.5 |
| L-threonine | 14 |
| L-valine | 71 |
| L-tyrosine | 167 |

This result indicates that valine and tyrosine are suitable as the 42nd amino acid.

In preparation of a mutant for the 92nd methionine, by using DNA 1 as a template, DNA amplified by PCR using two chemically-synthesized primer DNAs: 5'-ggggatcctcttgacaattagttaactatttgttataatgtattc-3' and 5'-tccgccgccaatcacrnngatttctggtacgtcacctgcg-3' (DNA 14) and DNA amplified by PCR using two chemically-synthesized primer DNAs: 5'-gacgtaccagaaatcnnygtgattggcggcggacgcgttt-3' and 5'-ggggatcccttatgcacagccaccgccaccacgacgctcgaggatttcg-3' (DNA15) were prepared. Subsequently, DNA14 was mixed with the same amount of DNA15 and then, DNA obtained through amplification by PCR using two chemically-synthesized primer DNAs: 5'-ggggatcctcttgacaattagttaactatttgttataatgtattc-3' and 5'-ggggatcccttatgcacagccaccgccaccacgacgctcgaggatttcg-3' (DNA 16) was prepared. After cleaving DNA16 with BamHI, the same procedures as described above were performed and colonies growing in agar medium 1 were inspected. The results are shown in Table 5.

**Table 5**

| [Types of mutation] | [DHFR activity] |
|---|---|
| (92nd amino acid) | |
| L-aspartic acid | 34 |
| L-phenylalanine | 69 |
| glycine | 0.2 |
| L-isoleucine | 37 |
| L-arginine | 0.5 |

This result indicates that phenylalanine and isoleucine are suitable as the 92nd amino acid.

Next, valine or tyrosine as the preferable 42nd amino acid of AS-DHFR-A1 was combined with phenylalanine or isoleucine as the preferable 92nd amino acid to prepare the 42nd and 92nd amino acid-substituted mutants of AS-DHFR-A1. The base sequence and the enzyme activity of the obtained mutants were measured.

Using pAS-DHFR-A1 as a template, DNA amplified by PCR using two chemically-synthesized primer DNAs: 5'-ggggatcctcttgacaattagttaactatttgttataatgtattc-3' and 5'-ccaggtatggcgcccrwmaatcacgggtttatttaaggtg-3' (r represents a base of a or g, w represents a base of a or t, and m represents a base of a or c, the same shall apply hereinafter) (DNA 17) and DNA amplified by PCR using two chemically-synthesized primer DNAs: 5'-aataaacccgtgattkwygggcgccatacctgggaatcaa-3' (k represents a base of g or t, w represents a base of a or t, and y represents a base of c or t, the same shall apply hereinafter) and 5'-ggggatcccttatgcacagccaccgccaccacgacgctcgaggatttcg-3' (DNA18) were prepared. Subsequently, DNA17 was mixed with the same amount of DNA18, and then, DNA was prepared by amplifying by PCR using two chemically-synthesized primer DNAs: 5'-ggggatcctcttgacaattagttaactatttgttataatgtattc-3' and 5'-ggggatcccttatgcacagccaccgccaccacgacgctcgaggatttcg-3' (DNA 19).

The obtained DNA19 was used as a template. DNA, amplified by PCR using two chemically-synthesized primer DNAs: 5'-ggggatcctcttgacaattagttaactatttgttataatgtattc-3' and 5-tccgccgccaatcacrawgatttctggtacgtcacctgcg-3', was prepared (DNA20) and DNA, amplified by PCR using two chemically-synthesized primer DNAs: 5'-gacgtaccagaaatcwtygtgattggcggcggacgcgttt-3' and 5'-ggggatcccttatgcacagccaccgccaccacgacgctcgaggatttcg-3'(DNA21), was prepared. Subsequently, DNA20 was mixed with the same amount of DNA21 and then DNA was prepared in which two chemically-synthesized primer DNAs: 5'-ggggatcctcttgacaattagttaactatttgttataatgtattc-3 and 5'-ggggatcccttatgcacagccaccgccaccacgacgctcgaggatttcg-3' were used and amplified by PCR (DNA 22).

After cleaving DNA22 with BamHI, the same procedures as described above were performed and colonies growing in agar medium 1 were inspected. The results are shown in Table 6.

**Table 6**

| [Types of mutation] | | [DHFR activity] |
|---|---|---|
| (42nd amino acid) | (92nd amino acid) | |
| L-tyrosine | L-phenylalanine | 320 |
| L-tyrosine | L-isoleucine | 61.3 |
| L-valine | L-phenylalanine | 102 |
| L-valine | L-isoleucine | 58.9 |

The result shows that a combination of tyrosine as the 42nd amino acid and phenylalanine as the 92nd amino acid exhibited the greatest activity (about three times greater than the wild type enzyme). This mutant is referred to as AS-DHFR-A I -M42Y-M92F. The recombinant plasmid including genes of AS-DHFR-A1-M42Y-M92F is named pAS-DHFR-A1-M42Y-M92F.

Alanine, phenylalanine, and asparagine as the preferable 16th amino acid of AS-DHFR-A1-M42Y-M92F were combined with isoleucine, leucine, and valine as the preferable 20th amino acid to prepare the 16th and 20the amino acid substitution mutants of AS-DHFR-A1-M42Y-M92F. The base sequence and the enzyme activity of the obtained mutants were measured.

DNA, amplified by PCR using three chemically-synthesized primer DNAs: 5'-ggggatcctcttgacaattagttaactatttgttataatgtattc-3', 5'-aggcaggttccacggrkwggcgttttcrytgccgataacgcgatctaccg-3', and 5'-aggcaggttccacggrkwggcgttttctgcgccgataacgcgatctaccg-3' (DNA23) and DNA, amplified by PCR using three chemically-synthesized primer DNAs: 5'-gatcgcgttatcggcarygaaaacgccwmyccgtggaacctgcctgccga-3', 5'-gatcgcgttatcggcgcagaaaacgccwmyccgtggaacctgcctgccga-3', and 5'-ggggatcccttatgcacagccaccgccaccacgacgctcgaggatttcg-3' (DNA24) were prepared using pAS-DHFR-Al-M42Y-M92F as a template. Subsequently, DNA23 was mixed with the same amount of DNA24. Thereafter, DNA, obtained through amplification by PCR using two chemically-synthesized primer DNAs: 5'-ggggatcctcttgacaattagttaactatttgttataatgtattc-3' and 5'-ggggatcccttatgcacagccaccgccaccacgacgctcgaggatttcg-3' was prepared (DNA25).

After cleaving DNA25 with BamHI, the same procedures as described above were performed and colonies growing in agar medium 1 were inspected. The results are shown in Table 7.

**Table 7**

| [Types of mutation] | | [DHFR activity] |
|---|---|---|
| (16th amino acid) | (20th amino acid) | |
| L-alanine | L-isoleucine | 240 |
| L-alanine | L-leucine | 579 |
| L-alanine | L-valine | 102 |
| L-phenylalanine | L-isoleucine | 176 |
| L-phenylalanine | L-leucine | 745 |
| L-phenylalanine | L-valine | 163 |
| L-asparagine | L-isoleucine | 102 |
| L-asparagine | L-leucine | 989 |
| L-asparagine | L-valine | 127 |

The results indicate that the combination of asparagine as the 16th and leucine as the 20th has the greatest activity (about 10 times greater than that of the wild type enzyme). This mutant is referred to as ANLYF. The recombinant plasmid including ANLYF genes was named pANLYF. Other 8 mutants had activities exceeding the wild type enzyme. This result indicates that there are many possibilities for sulfur atom-free enzyme proteins.

SEQ ID NO 5 in the Sequence Listing shows the amino acid sequence of ANLYF and SEQ ID NO 6 in the Sequence Listing shows the ANLYF gene sequence which is excisable by restriction enzyme BamHI and highly expressible in Escherichia coli by introduction thereof into a BamHI site with an appropriate vector.

Escherichia coli containing pANLYF was cultured in 3L of medium (containing 15 g of salt, 15 g of yeast extract, 24 g of trypton, and 30 mg of sodium ampicillin) at 37°C overnight to obtain about 10 g of cells on a wet basis. A cell-free extract from the cells was subjected to purification by streptomycin sulfate treatment, ammonium sulfate fractionation, methotrexate affinity chromatography, and DEAE Toyopearl chromatography to purify proteins to homogeneity. Thus, about 100 mg of homogeneous ANLYF was obtained. As a result of N-terminal analysis, the amino-terminal sequence of ANLYF was L-alanine-L-isoleucine-L-serine-L-leucine-L-isoleucine and was a sequence with an initiation codon which is corresponding to L-methionine being removed therefrom. 1 mg of ANLYF obtained through purification was allowed to stand overnight at room temperature in 10 mM phosphate buffer (pH 7.0) containing 0.1M hydrogen peroxide water. Molecular weight was measured, and as a result, the measured value was exactly same as the molecular weight of untreated ANLYF, i.e., 17,905 (calculated value: 17,903). Enzyme activity was also completely unchanged. In contrast, AS-DHFR was subjected to the same treatment, and as a result, molecular weight before treatment with hydrogen peroxide water was 17,954 (calculated value: 17,950) and the molecular weight after treatment with hydrogen peroxide water was 18,034. This indicates that all methionine residues were oxidized to methionine sulfoxide. The enzyme activity of AS-DHFR was lowered to about one fifth as a result of oxidation of all methionine residues. Thus, an antioxidative property was imparted by the exclusion of sulfur atoms.

### [Example 2] Preparation of sulfur atom-free dihydrofolate reductase-xylanase fusion enzyme

For the preparation of a fused gene of dihydrofolate reductase and xylanase, as a gene portion of dihydrofolate reductase, a sequence corresponding to 1 to positions No. 1 to No. 560 in SEQ ID NO 6 of the Sequence Listing was used. As a xylanase gene portion, DNA was prepared by amplifying by PCR using two chemically-synthesized primer DNAs: 5'-gctagcacag actactggcaaaat-3' and 5'-ttaccatacggtaacattcgacg-3' (DNA26) and using chromosome DNA as a template, which is commercially available from Sigma as chromosome DNA of Bacillus subtilis (product number D4041). The prepared DNA was linked with a base sequence corresponding to the codon for Gly-Gly-Gly-Gly-Ser-Gly-Gly-Gly-Gly to prepare a fusion gene.

At the outset, DNA was prepared by PCR amplification using two chemically-synthesized primer DNAs: 5'-ggggatcctcttgacaattagttaactatttgttataatgtattc-3' and 5'-gccgccaccacccgagccaccgccaccacgacgctcgaggatttcgaacgaata-3', and using pNALYF as a template (DNA27). Subsequently, DNA26 was as a template to prepare DNA by PCR amplification using two chemically-synthesized primer DNAs: 5'-ggtggcggtggctcgggtggtggcggcgctagcacagactactggcaaaattggactgat-3' and 5'-ggggatccttaccatacggtaacattcgacgagccactactttga-3' (DNA28). DNA27 was mixed with the same amount of DNA28, thereafter, two chemically-synthesized primer DNAs: 5'-ggggatcctcttgacaattagttaactatttgttataatgtattc-3' and 5'-ggggatccttaccatacggtaacattcgacgagccactactttga-3' were used to prepare DNA obtained through amplification by PCR (DNA29).

DNA29 was cleaved by BamHI, and then, 10 colonies growing in agar medium 1 were collected in the same manner as described above, a recombinant plasmid was isolated, the base sequence of the portion included in the plasmid inspected, and a recombinant plasmid which included the subject sequence was isolated, and named pNLXYL-wt.

SEQ ID NO: 7 of the Sequence Listing shows the amino acid sequence of dihydrofolate reductase-xylanase fusion enzyme. SEQ ID NO: 8 of the Sequence Listing shows the gene sequence of dihydrofolate reductase-xylanase fusion enzyme which can be cleaved by restriction enzyme BamHI and is highly expressible in Escherichia coli by introduction thereof into a BamHI site with an appropriate vector. A sequence corresponding to positions No. 1 to No. 159 in SEQ ID NO 7 in the Sequence Listing is the dihydrofolate reductase (ANLYF mutant) portion, the sequence corresponding to positions No. 160 to No. 168 is a linker sequence for linking dihydrofolate reductase to xylanase without difficulties, and the sequence corresponding to positions No. 169 to No. 353 is a sequence of the wild type xylanase.

Xylanase comprises 185 amino acids. Among them, the 158th and the 169th amino acids are methionine. Thus, in dihydrofolate reductase-xylanase fused enzyme, the 326th and the 337th sequences are methionines. In this dihydrofolate reductase-xylanase fusion enzyme, no other sulfur-containing amino acids exist besides the above.

In preparation of a 326th methionine mutant of a dihydrofolate reductase-xylanase fusion enzyme, pNLXYL-wt was used as a template to prepare DNA amplified by PCR using two chemically-synthesized primer DNAs: 5'-ggggatcctcttgacaattagttaactatttgttataatgtattc-3' and 5'-gacttggtaagcccaattactgcccagattgnntccatggctcttccatgcgtt-3' (DNA30). DNA30 was used as a template to prepare DNA amplified by PCR using two chemically-synthesized primer DNAs: 5'-ggggatcctcttgacaattagttaactatttgttataatgtattc-3' and 5'-actttgatatccttctgtcgccatgacttggtaagcccaattactgcccagatt-3' (DNA31). Further, DNA31 was used as a template to prepare DNA amplified by PCR using two chemically-synthesized primer DNAs: 5'-ggggatcctcttgacaattagttaactatttgttataatgtattc-3' and 5'-ggggggatccttaccatacggtaacattcgacgagccactactttgatatccttctgtcgc-3' (DNA32). After cleaving DNA32 with BamHI, the same procedures as described above were performed and colonies growing in agar medium 1 were inspected. The xylanase activity of each mutant was also inspected.

From the measured xylanase activity and dihydrofolate reductase activity, the value [xylanase activity]/[DHFR activity] was calculated, and compared to value [xylanase activity]/[DHFR activity] exhibited by cells, which retain pNLXYL-wt (this is determined as the activity of the wild type enzyme). The results are shown in Table 8. In the following table, [xylanase activity]/[DHFR activity] is indicated by % with the activity of the wild type enzyme being 100%.

**Table 8**

| [Type of mutation] | [xylanase activity]/[DHFR activity] |
|---|---|
| (326th amino acid) | |
| L-aspartic acid | 0.0 |
| L-phenylalanine | 112 |
| L-histidine | 5.9 |
| L-isoleucine | 66 |
| L-leucine | 127 |
| L-asparagine | 100 |
| L-serine | 7.9 |
| L-threonine | 0.7 |
| L-valine | 58 |

This result indicates that leucine is suitable as the 326th amino acid.

This mutant was named NL-xylanase (M158L). A recombinant plasmid which includes NL-xylanase (M158L) gene was named pNLXYL-M158L.

In preparation of a 337th methionine mutant of NL-xylanase (M158L), pNLXYL-M158L was used as a template, and DNA was prepared by amplifying by PCR using two chemically-synthesized primer DNAs: 5'-ggggatcctcttgacaattagttaactatttgttataatgtattc-3' and 5'-actttgatatccttctgtcgcgnngacttggtaagcccaattactgcccagatt-3' (DNA33). DNA33 was used as a template and DNA was prepared by amplifying by PCR using two chemically-synthesized primer DNAs: 5'-ggggatcctcttgacaattagttaactatttgttataatgtattc-3' and 5'-ggggggatccttaccatacggtaacattcgacgagccactactttgatatccttctgtcgc-3' (DNA34). After cleaving DNA34 with BamHI, the same procedures as described above were performed and colonies growing in agar medium 1 were inspected. The xylanase activity of each mutant was also inspected. The results are shown in Table 9.

**Table 9**

| [Type of mutation] | [Xylanase activity]/[DHFR activity] |
|---|---|
| (337th amino acid) | |
| L-aspartic acid | 0.1 |
| Glycine | 0.0 |
| L-histidine | 111 |
| L-isoleucine | 151 |
| L-asparagine | 26 |
| L-proline | 1.7 |
| L-arginine | 0.0 |
| L-threonine | 0.7 |
| L-tyrosine | 2.1 |

This result indicates that isoleucine is suitable as the 326th amino acid.

This mutant was named NL-xylanase (M158L, M169I). A recombinant plasmid which includes NL-xylanase (M158L, M169I) genes was named pNLXYL-LI.

SEQ ID NO 9 in the Sequence Listing shows NL-xylanase (M158L, M169I), and SEQ ID NO 10 in the Sequence Listing shows NL-xylanase (M158L, M169I) which can be cleaved by restriction enzyme BamHI and is highly expressible in Escherichia coli by introduction into a BamHI site with an appropriate vector.

Escherichia coli containing pNLXYL-LI was cultured in 3 L of medium (containing 15 g of salt, 15 g of yeast extract, 24 g of trypton, and 30 mg of sodium ampicillin) at 37°C overnight to obtain about 10 g of cells on a wet basis. Cell-free extract from the cells was subjected to purification by a streptomycin sulfuric acid treatment, ammonium sulfate fraction, methotrexate affinity chromatography, and DEAE Toyopearl chromatography to purify proteins to homogeneity. Thus, about 30 mg of homogeneous NL-xylanase (M158L, M1691) was obtained. As a result of N-terminal analysis, amino-terminal sequence of NL-xylanase (M158L, M169I) was L-alanine-L-isoleucine-L-serine-L-leucine-L-isoleucine-and was a sequence from which an initiation codon which is corresponding to L-methionine had been removed One mg of NL-xylanase (M158L, M169I) obtained through purification was allowed to stand overnight at room temperature in 10 mM phosphate buffer (pH 7.0) containing 0.1M hydrogen peroxide water. Molecular weight was measured, and as a result, the measured value was exactly same as the molecular weigh of untreated NL-xylanase (M158L, M169I), i.e., 38,775 (calculated value: 38,773). The enzyme activity was also completely unchanged. In contrast, dihydrofolate reductase-xylanase fused enzyme before mutation was subjected to the same treatment, and as a result, the molecular weight before treatment with hydrogen peroxide water was 38,813 (calculated value: 38,809) and the molecular weight after treatment with hydrogen peroxide water was 38,845. This indicates that all methionine residues were oxidized to methionine sulfoxide. The enzyme activity of xylanase was lowered to about one third as a result of oxidation of all methionine residues. Thus, an antioxidative property was imparted by the exclusion of sulfur atoms.

### INDUSTRIAL APPLICABILITY

The present invention provides a method for producing enzyme proteins which retain the activity of the wild type enzyme while haying resistance against oxidation caused by hydrogen peroxide and the like, and being chemically stable. The enzyme proteins have stable properties, and thus, can be widely used in applications such as biosensors and bioreactors. Therefore, the enzyme proteins are valuable from a practical point of view.

## Claims

1. A method of producing a sulfur atom-free enzyme protein having activity greater than or equivalent to the original enzyme protein from which said sulfur atom-free enzyme protein is derived, comprising the following steps:
(1) preparing respective mutant genes by substituting an initiation codon encoding L-methionine in a DNA sequence encoding an enzyme protein which consists of a total length of m amino acids and which comprises n sulfur atom-containing amino acids (sulfur-containing amino acids), wherein the respective sulfur-containing amino acids in the sequence are designated Ai (i = 1 to n), by codons for L-methionine-L-alanine, L-methionine-L-serine, or L-methionine-L-proline; expressing each mutant gene in a host cell; measuring enzyme activity of each obtained mutant enzyme protein; and selecting the protein with the highest activity, thereby obtaining a substitution mutant A1/MA1;
(2) preparing respective mutant genes in which a codon encoding a sulfur-containing amino acid at another site Ai (i = 2 to n) is substituted with a codon encoding another amino acid selected from L-alanine, L-aspartic acid, L-glutamic acid, L-phenylalanine, L-glycine, L-histidine, L-isoleucine, L-lysine, L-leucine, L-asparagine, L-proline, L-glutamine, L-arginine, L-serine, L-threonine, L-valine, L-tyrosine, and L-tryptophan; expressing each mutant gene in a host cell; measuring the enzyme activity of each obtained mutant enzyme protein; and selecting p mutant enzyme proteins having enzyme activity, thereby obtaining substitution mutants Ai/Bij (j = 1 to p);
(3) selecting from the p mutant enzyme proteins produced in (2) a maximum of 3 substitution mutants having the highest activity Ai/Bi1, Ai/Bi2, and Ai/Bi3, wherein activity decreases in order, Ai/Bi1>Ai/Bi2>Ai/Bi3>..>Ai/Bip; and
(4) selecting according to steps (2) and (3) for all sites Ai (i = 2 to n) a maximum of 3 substitution mutants having activity; preparing a maximum of 3 x (n-1) mutants which constitute all combinations of the selected substitution mutants with the mutant of A1/MA1, measuring the enzyme activity thereof, and obtaining a mutant enzyme protein having activity greater than or equivalent to the original enzyme protein.

2. A method of producing a sulfur atom-freeenzyme protein having activity greater than or equivalent to the original enzyme protein from which said sulfur atom-free enzyme protein is derived, comprising the following steps:
(1) preparing respective mutant genes by substituting an initiation codon encoding L-methionine in a DNA sequence encoding an enzyme protein which consists of a total length of m amino acids and which comprises n sulfur atom-containing amino acids (sulfur-containing amino acids), wherein the respective sulfur-containing amino acids in the sequence are designated Ai (i = 1 to n), by codons for L-methionine-L-alanine, L-methionine-L-serine or L-methionine-L-proline; expressing each mutant gene in a host cell; measuring enzyme activity of each obtained mutant enzyme protein; and selecting the protein with the highest activity, thereby obtaining a substitution mutant A1/MA1;
(2) preparing respective mutant genes in which a codon encoding a sulfur-containing amino acid at positions A2 of the A1/MA1 mutant is substituted with a codon encoding another amino acid selected from L-alanine, L-aspartic acid, L-glutamic acid, L-phenylalanine, L-glycine, L-histidine, L-isoleucine, L-lysine, L-leucine, L-asparagine, L-proline, L-glutamine, L-arginine, L-serine, L-threonine, L-valine, L-tyrosine and L-tryptophan; expressing each mutant gene in a host cell, measuring the enzyme activity of each obtained double mutant enzyme protein; and selecting a maximum of 3 double mutants with the highest activity;
(3) preparing respective mutant genes in which a codon encoding a sulfur-containing amino acid at position A3 of each obtained double mutant is substituted with a codon encoding another amino acid selected from L-alanine, L-aspartic acid, L-glutamic acid, L-phenylalanine, L-glycine, L-histidine, L-isoleucine, L-lysine, L-leucine, L-asparagine, L-proline, L-glutamine, L-arginine, L-serine, L-threonine, L-valine, L-tytosine and L-tryptophan; expressing each mutant gene in a host cell; measuring the enzyme activity of each obtained triple mutant enzyme protein; and selecting a maximum of 3 triple mutants with the highest activity; and
(4) preparing mutants in the same manner as described above at each further site of a sulfur-containing amino acid up to site n; inspecting the enzyme activity of the mutants produced at site n; and selecting a mutant enzyme protein having activity greater than or equivalent to that of the original enzyme.

3. A method according to claim 2 wherein the order of stepwise mutation sites is according to any one of n! permutations and combinations of A1, A2, ..., An.

4. A method for producing a sulfur atom-free enzyme protein having activity greater than or equivalent to the original enzyme protein from which said sulfur atom-free enzyme protein is derived, wherein, in an enzyme protein consisting of a total length of m amino acids and comprising n sulfur-containing amino acids and in which the position of a sulfur-containing amino acid in the sequence is designated Ai (i = 1 to n), the method according to claim 1 is adopted at k sites and the method according to claim 2 or 3 is adopted at n- k sites wherein k is a natural number less than n.

5. A method according to any one of the preceding claims wherein the host cell is *Escherichia coli.*

6. A method according to any one of the preceding claims wherein amino acid substitution at a specific site in an enzyme protein is carried out by site-directed mutagenesis using synthetic DNA.

7. A method according to any one of the preceding claims wherein the sulfur atom-free enzyme protein is *E. coli*-derived dihydrofolate reductase.

8. A method according to any one of claims 1 to 6 wherein the sulfur atom-free enzyme protein is *B. subtilis*-derived xylanase.

## Patentansprüche

1. Verfahren zur Herstellung eines Schwefelatom-freien Enzymproteins, das eine höhere oder gleiche Aktivität wie diejenige des ursprünglichen Enzymproteins aufweit, von dem sich das Schwefelatom-freie Enzymprotein ableitet, umfassend die folgenden Schritte:
(1) Herstellung der jeweiligen mutierten Gene durch Substituieren eines L-Methionin codierenden Startcodons in einer DNA-Sequenz, die ein Enzymprotein codiert, das aus einer Gesamtlänge von m Aminosäuren besteht und das n Schwefelatom-enthaltende Aminosäuren (schwefelhaltige Aminosäuren) umfasst, wobei die jeweiligen schwefelhaltigen Aminosäuren in der Sequenz als Ai (i = 1 bis n) bezeichnet sind, durch Codons für L-Methionin-L-Alanin, L-Methionin-L-Serin oder L-Methionin-L-Prolin; Exprimieren jedes mutierten Gens in einer Wirtszelle; Messen der Enzymaktivität jedes erhaltenen mutierten Enzymproteins; und Auswählen des Proteins mit der höchsten Aktivität, wodurch eine Substitutionsmutante A1/MA1 erhalten wird;
(2) Herstellen der jeweiligen mutierten Gene, in denen ein Codon, das eine schwefelhaltige Aminosäure an einer anderen Stelle Ai (i = 2 bis n) codiert, mit einem Codon, das eine andere Aminosäure ausgewählt aus L-Alanin, L-Asparaginsäure, L-Glutaminsäure, L-Phenylalanin, L-Glycin, L-Histidin, L-Isoleucin, L-Lysin, L-Leucin, L-Asparagin, L-Prolin, L-Glutamin, L-Arginin, L-Serin, L-Threonin, L-Valin, L-Tyrosin und L-Tryptophan codiert, substituiert ist; Exprimieren jedes mutierten Gens in einer Wirtszelle; Messen der Enzymaktivität jedes erhaltenen mutierten Enzymproteins; und Auswählen von p mutierten Enzymproteinen mit enzymatischer Aktivität, wodurch die Substitutionsmutanten Ai/Bij (j = 1 bis p) erhalten werden;
(3) Auswählen von höchstens 3 Substitutionsmutanten mit der höchsten Aktivität Ai/Bi1, Ai/Bi2 und Ai/Bi3 unter den in (2) erzeugten p mutierten Enzymproteinen, wobei die Aktivität in der Reihenfolge Ai/Bil>Ai/Bi2>Ai/Bi3>..>Ai/ Bip abnimmt; und
(4) Auswählen von höchstens 3 Substitutionsmutanten mit Aktivität gemäß den Schritten (2) und (3) für alle Stellen Ai (i = 2 bis n); Herstellen von höchstens 3 x (n-1) Mutanten, welche alle Kombinationen der ausgewählten Substitutionsmutanten mit der Mutante A1/MA1 bilden ; Messen der Enzymaktivität davon; und Erhalten eines mutierten Enzymproteins, das eine höhere oder gleiche Aktivität wie diejenige des ursprünglichen Enzymproteins aufweist.

2. Verfahren zur Herstellung eines Schwefelatom-freien Enzymproteins, das eine höhere oder gleiche Aktivität als diejenige des ursprünglichen Enzymproteins aufweist, von dem sich das Schwefelatom-freie Enzymprotein ableitet, umfassend die folgenden Schritte:
(1) Herstellen der jeweiligen mutierten Gene durch Substituieren eines L-Methionin codierenden Startcodons in einer DNA-Sequenz, die ein Enzymprotein codiert, das aus einer Gesamtlänge von m Aminosäuren besteht und das n Schwefelatom-enthaltende Aminosäuren (schwefelhaltige Aminosäuren) umfasst, wobei die jeweiligen schwefelhaltigen Aminosäuren in der Sequenz als Ai (i = 1 bis n) bezeichnet sind, durch Codons für L-Methionin-L-Alanin, L-Methionin-L-Serin oder L-Methionin-L-Prolin; Exprimieren jedes mutierten Gens in einer Wirtszelle; Messen der Enzymaktivität jedes erhaltenen mutierten Enzymproteins; und Auswählen des Proteins mit der höchsten Aktivität, wodurch eine Substitutionsmutante A1/MA1 erhalten wird;
(2) Herstellen der jeweiligen mutierten Gene, in denen ein Codon, das eine schwefelhaltige Aminosäure an der Stelle A2 der A1/MA1-Mutante codiert, mit einem Codon, das eine andere Aminosäure ausgewählt aus L-Alanin, L-Asparaginsäure, L-Glutaminsäure, L-Phenylalanin, L-Glycin, L-Histidin, L-Isoleucin, L-Lysin, L-Leucin, L-Asparagin, L-Prolin, L-Glutamin, L-Arginin, L-Serin, L-Threonin, L-Valin, L-Tyrosin und L-Tryptophan codiert, substituiert ist; Exprimieren jedes mutierten Gens in einer Wirtszelle; Messen der Enzymaktivität jedes erhaltenen doppelmutierten Enzymproteins; und Auswählen von höchstens 3 Doppelmutanten mit der höchsten Aktivität;
(3) Herstellen der jeweiligen mutierten Gene, in denen ein Codon, das eine schwefelhaltige Aminosäure an der Position A3 jeder erhaltenen Doppelmutante codiert, mit einem Codon, das eine andere Aminosäure ausgewählt aus L-Alanin, L-Asparaginsäure, L-Glutaminsäure, L-Phenylalanin, L-Glycin, L-Histidin, L-Isoleucin, L-Lysin, L-Leucin, L-Asparagin, L-Prolin, L-Glutamin, L-Arginin, L-Serin, L-Threonin, L-Valin, L-Tyrosin und L-Tryptophan codiert, substituiert ist; Exprimieren jedes mutierten Gens in einer Wirtszelle; Messen der Enzymaktivität jedes erhaltenen dreifach mutierten Enzymproteins; und Auswählen von höchstens 3 Dreifachmutanten mit der höchsten Aktivität; und
(4) Herstellen von Mutanten auf dieselbe Art und Weise wie oben beschrieben an jeder weiteren Stelle einer schwefelhaltigen Aminosäure bis zur Stelle n; Untersuchen der Enzymaktivität der an der Stelle n erzeugten Mutanten; und Auswählen eines mutierten Enzymproteins, das eine höhere oder gleiche Aktivität wie diejenige des ursprünglichen Enzyms aufweist.

3. Verfahren nach Anspruch 2, wobei die Reihenfolge der stufenweisen Mutationsstellen irgendeiner von n! Permutationen und Kombinationen von A1, A2, ..., An folgt.

4. Verfahren zur Herstellung eines Schwefelatom-freien Enzymproteins, das eine höhere oder gleiche Aktivität wie diejenige des ursprünglichen Enzymproteins aufweist, von dem sich das Schwefelatom-freie Enzymprotein ableitet, wobei in einem Enzymprotein, das aus einer Gesamtlänge von m Aminosäuren besteht und n schwefelhaltige Aminosäuren umfasst und in dem die Position einer schwefelhaltigen Aminosäure in der Sequenz als Ai (i = 1 bis n) bezeichnet ist, das Verfahren nach Anspruch 1 an k Stellen angewendet wird und das Verfahren nach Anspruch 2 oder 3 an n-k Stellen angewendet wird, wobei k eine natürliche Zahl kleiner als n ist.

5. Verfahren nach einem der vorhergehenden Ansprüche, wobei die Wirtszelle *Escherichia coli* ist.

6. Verfahren nach einem der vorhergehenden Ansprüche, wobei die Aminosäuresubstitution durch ortsspezifische Mutagenese an einer bestimmten Stelle in einem Enzymprotein unter Verwendung einer synthetischen DNA durchgeführt wird.

7. Verfahren nach einem der vorhergehenden Ansprüche, wobei das Schwefelatom-freie Enzymprotein eine *E*. *coli*-abgeleitete Dihydrofolatreduktase ist.

8. Verfahren nach einem der Ansprüche 1 bis 6, wobei das Schwefelatom-freie Enzymprotein eine *B. subtilis-*abgeleitete Xylanase ist.

## Revendications

1. Procédé de production d'une protéine enzymatique sans atome de soufre ayant une activité supérieure ou équivalente à celle de la protéine enzymatique d'origine dont ladite protéine enzymatique sans atome de soufre est dérivée, comprenant les étapes suivantes consistant à :
(1) préparer des gènes mutants respectifs en remplaçant un codon d'initiation codant pour la L-méthionine dans une séquence d'ADN codant pour une protéine enzymatique qui est constituée d'une longueur totale de m acides aminés et qui comprend n acides aminés contenant un atome de soufre (acides aminés contenant du soufre), lesdits acides aminés contenant du soufre respectifs présents dans la séquence étant désignés par Ai (i = 1 à n), par des codons pour L-méthionine-L-alanine, L-méthionine-L-sérine ou L-méthionine-L-proline ; exprimer chaque gène mutant dans une cellule hôte ; mesurer l'activité enzymatique de chaque protéine enzymatique mutante obtenue ; et sélectionner la protéine ayant l'activité la plus élevée, ce par quoi on obtient un mutant par substitution A1/MA1 ;
(2) préparer des gènes mutants respectifs dans lesquels un codon codant pour un acide aminé contenant du soufre au niveau d'un autre site Ai (i = 2 à n) est remplacé par un codon codant pour un autre acide aminé choisi entre la L-alanine, l'acide L-aspartique, l'acide L-glutamique, la L-phénylalanine, la L-glycine, la L-histidine, la L-isoleucine, la L-lysine, la L-leucine, la L-asparagine, la L-proline, la L-glutamine, la L-arginine, la L-sérine, la L-thréonine, la L-valine, la L-tyrosine et le L-tryptophane ; exprimer chaque gène mutant dans une cellule hôte ; mesurer l'activité enzymatique de chaque protéine enzymatique mutante obtenue ; et sélectionner p protéines enzymatiques mutantes ayant une activité enzymatique, ce par quoi on obtient des mutants par substitution Ai/Bij (j = 1 à p) ;
(3) sélectionner parmi les p protéines enzymatiques mutantes produites en (2) un maximum de 3 mutants par substitution ayant l'activité la plus élevée Ai/Bi1, Ai/Bi2 et Ai/Bi3, avec une activité diminuant dans l'ordre Ai/Bi1 > Ai/Bi2 > Ai/Bi3 >...> Ai/Bip ; et
(4) sélectionner selon les étapes (2) et (3) pour tous les sites Ai (i = 2 à n) un maximum de 3 mutants par substitution ayant une activité ; préparer un maximum de 3 x (n - 1) mutants qui constituent toutes les combinaisons des mutants par substitution sélectionnés avec le mutant A1/MA1 ; mesurer l'activité enzymatique de ceux-ci ; et obtenir une protéine enzymatique mutante ayant une activité supérieure ou équivalente à celle de la protéine enzymatique d'origine.

2. Procédé de production d'une protéine enzymatique sans atome de soufre ayant une activité supérieure ou équivalente à celle de la protéine enzymatique d'origine dont ladite protéine enzymatique sans atome de soufre est dérivée, comprenant les étapes suivantes consistant à :
(1) préparer des gènes mutants respectifs en remplaçant un codon d'initiation codant pour la L-méthionine dans une séquence d'ADN codant pour une protéine enzymatique qui est constituée d'une longueur totale de m acides aminés et qui comprend n acides aminés contenant un atome de soufre (acides aminés contenant du soufre), lesdits acides aminés contenant du soufre respectifs présents dans la séquence étant désignés par Ai (i = 1 à n), par des codons pour L-méthionine-L-alanine, L-méthionine-L-sérine ou L-méthionine-L-proline ; exprimer chaque gène mutant dans une cellule hôte ; mesurer l'activité enzymatique de chaque protéine enzymatique mutante obtenue ; et sélectionner la protéine ayant l'activité la plus élevée, ce par quoi on obtient un mutant par substitution A1/MA1 ;
(2) préparer des gènes mutants respectifs dans lesquels un codon codant pour un acide aminé contenant du soufre en position A2 du mutant A1/MA1 est remplacé par un codon codant pour un autre acide aminé choisi entre la L-alanine, l'acide L-aspartique, l'acide L-glutamique, la L-phénylalanine, la L-glycine, la L-histidine, la L-isoleucine, la L-lysine, la L-leucine, la L-asparagine, la L-proline, la L-glutamine, la L-arginine, la L-sérine, la L-thréonine, la L-valine, la L-tyrosine et le L-tryptophane ; exprimer chaque gène mutant dans une cellule hôte ; mesurer l'activité enzymatique de chaque protéine enzymatique doublement mutante obtenue ; et sélectionner un maximum de 3 doubles mutants ayant l'activité la plus élevée ;
(3) préparer des gènes mutants respectifs dans lesquels un codon codant pour un acide aminé contenant du soufre en position A3 de chaque double mutant obtenu est remplacé par un codon codant pour un autre acide aminé choisi entre la L-alanine, l'acide L-aspartique, l'acide L-glutamique, la L-phénylalanine, la L-glycine, la L-histidine, la L-isoleucine, la L-lysine, la L-leucine, la L-asparagine, la L-proline, la L-glutamine, la L-arginine, la L-sérine, la L-thréonine, la L-valine, la L-tyrosine et le L-tryptophane ; exprimer chaque gène mutant dans une cellule hôte ; mesurer l'activité enzymatique de chaque protéine enzymatique triplement mutante obtenue ; et sélectionner un maximum de 3 triples mutants ayant l'activité la plus élevée ; et
(4) préparer des mutants de la même manière que celle décrite ci-dessus au niveau de chaque site supplémentaire d'un acide aminé contenant du soufre jusqu'au site n ; examiner l'activité enzymatique des mutants produits au niveau du site n ; et sélectionner une protéine enzymatique mutante ayant une activité supérieure ou équivalente à celle de la protéine enzymatique d'origine.

3. Procédé selon la revendication 2 dans lequel l'ordre des sites de mutation par étapes est selon l'une quelconque des n! permutations et combinaisons de A1, A2,..., An.

4. Procédé de production d'une protéine enzymatique sans atome de soufre ayant une activité supérieure ou équivalente à celle de la protéine enzymatique d'origine dont ladite protéine enzymatique sans atome de soufre est dérivée, dans lequel, dans une protéine enzymatique constituée d'une longueur totale de m acides aminés et comprenant n acides aminés contenant du soufre et dans laquelle la position d'un acide aminé contenant du soufre présent dans la séquence est désignée par Ai (i = 1 à n), le procédé selon la revendication 1 est adopté au niveau de k sites et le procédé selon la revendication 2 ou 3 est adopté au niveau de n-k sites, k étant un nombre entier naturel inférieur à n.

5. Procédé selon l'une quelconque des revendications précédentes dans lequel la cellule hôte est *Escherichia coli.*

6. Procédé selon l'une quelconque des revendications précédentes dans lequel la substitution d'acides aminés au niveau d'un site spécifique dans une protéine enzymatique est effectuée par mutagenèse dirigée en utilisant un ADN synthétique.

7. Procédé selon l'une quelconque des revendications précédentes dans lequel la protéine enzymatique sans atome de soufre est une dihydrofolate réductase dérivée de *E. coli.*

8. Procédé selon l'une quelconque des revendications 1 à 6 dans lequel la protéine enzymatique sans atome de soufre est une xylanase dérivée de *B. subtilis*
